# EUROPEAN PATENT APPLICATION

(11) **EP 4 814 114 A1**
(43) Date of publication of application: **30.09.2026**
(21) Application number: 25741395.5
(22) Date of filing: 09.01.2025
(51) Int. Cl.: C12N 9/12

(54) **RNA POLYMERASE VARIANT AND USE THEREOF**

(30) Priority: 19.01.2024 CN 202410080996; 17.06.2024 CN 202410777724
(71) Applicant: Nanjing Vazyme Biotech Co., Ltd., Nanjing, Jiangsu 210046 (CN)
(72) Inventor: XU, Xiaoyu, Nanjing, Jiangsu 210046 (CN); JIN, Qiuheng, Nanjing, Jiangsu 210046 (CN); HE, Wei, Nanjing, Jiangsu 210046 (CN); JI, Guiying, Nanjing, Jiangsu 210046 (CN); GENG, Qi, Nanjing, Jiangsu 210046 (CN); SUN, Yujian, Nanjing, Jiangsu 210046 (CN); ZHANG, Xinya, Nanjing, Jiangsu 210046 (CN); ZHOU, Hongli, Nanjing, Jiangsu 210046 (CN)
(74) Representative: Ostertag & Partner Patentanwälte mbB
(86) International application number: PCT/CN2025/071395
(87) International publication number: WO 2025/152833

(57) **Abstract**

Provided are an RNA polymerase variant and a preparation method therefor. The RNA polymerase variant is used in *in vitro* transcription to obtain an RNA product having low dsRNA contamination. In addition, provided is a method for preparing an RNA by means of *in vitro* transcription.

## Description

### Technical Field

The present application belongs to the field of biotechnology, and specifically relates to an RNA polymerase variant, a preparation method therefor, and the use thereof in RNA synthesis.

### Background Art

At the end of 2019, Corona Virus Disease 2019 (COVID-19) broke out globally, infecting billions of people worldwide. Before the launch of Paxlovid (nirmatrelvir/ritonavir combination pack), which received emergency use authorization from the FDA on 22 November 2021, vaccines served as the most effective line of defence against infection. During this massive global fight against the pandemic, billions of people received COVID-19 vaccines, including BNT162b2 (an mRNA vaccine) developed by BioNTech and Pfizer, mRNA-1273 (an mRNA vaccine) developed by Moderna, USA, AZD1222 (an adenovirus vector vaccine) developed by AstraZeneca, etc. Among these, RNA vaccines exhibited the highest protective efficacy. By preventing infection, reducing the incidence of severe cases, and curbing the spread of the pandemic, mRNA vaccines have made tremendous contributions to this fight against the pandemic.

mRNA vaccines have a relatively short research and development cycle, enabling rapid development of novel vaccine candidates to address viral mutations. With dual mechanisms of humoral immunity and T-cell immunity, they exhibit strong immunogenicity and remarkable efficacy. Furthermore, their production process is simple, which facilitates efficient research and development and large-scale production, thus allowing rapid global supply for responses to pandemics similar to the COVID-19 crisis.

In addition to mRNA, breakthrough progress has also been made in the research on circular RNA (circRNA)-related drugs. Orna Therapeutics has developed *in vivo* cell therapy products using circRNA. In a research report presented at the 2022 Annual Meeting of the American Society of Gene and Cell Therapy (ASGCT), the company has demonstrated that these products also hold great application potential in tumour therapy and other fields.

RNA has shown great promise in the research and development of vaccines and other drugs. However, the removal of certain impurities during actual production requires further investigation. In particular, double-stranded RNA (dsRNA) impurities can induce strong immunogenicity (Goubau et al., 2014; Kato et al., 2006; Mu et al., 2018). Therefore, there is an urgent need to develop a method for effectively reducing dsRNA impurities.

### Summary of the Invention

In a first aspect, the present application provides an RNA polymerase variant, wherein the amino acid sequence of the variant, compared with SEQ ID NO: 1, comprises at least one mutation at amino acid positions selected from the following: R34, R52, F55, L59, I154, L170, K172, R173, M183, E187 and G753, wherein the mutation type is selected from a substitution or deletion.

In a second aspect, the present application provides a biological material selected from one or more of the following:
1) a polynucleotide molecule encoding the above variant;
2) an expression vector comprising the polynucleotide molecule of 1); and
3) a host cell comprising the polynucleotide molecule of 1) or a host cell comprising the expression vector of 2).

In a third aspect, the present application provides a method for preparing the above RNA polymerase variant.

In a fourth aspect, the present application provides a composition comprising at least one RNA polymerase variant of the present application.

In a fifth aspect, the present application provides a kit comprising at least one RNA polymerase variant of the present application.

In a sixth aspect, the present application provides the use of the above RNA polymerase variant, composition or kit in the preparation of RNA by *in vitro* transcription.

In a seventh aspect, the present application further provides a method for preparing RNA.

### Detailed Description of the Invention

### RNA polymerase variant

In a first aspect, the present application provides an RNA polymerase variant, wherein the amino acid sequence of the variant, compared with SEQ ID NO: 1, comprises at least one mutation at amino acid positions selected from the following: R34, R52, F55, L59, I154, L170, K172, R173, M183, E187 and G753, wherein the mutation type is selected from a substitution or deletion.

In some embodiments, the amino acid sequence of the variant has one amino acid mutation relative to SEQ ID NO: 1, and the position of the mutated amino acid is selected from R52, F55, L59, I154, L170, K172, M183, E187 and G753.

In some embodiments, the mutation at position R52 is R52A;
in some embodiments, the mutation at position F55 is selected from F55E, F55G, F55K, F55L or F55N;
in some embodiments, the mutation at position L59 is L59A;
in some embodiments, the mutation at position I154 is I154D;
in some embodiments, the mutation at position L170 is selected from L170A or L170D;
in some embodiments, the mutation at position K172 is a deletion;
in some embodiments, the mutation at position M183 is M183E;
in some embodiments, the mutation at position E187 is E187D;
in some embodiments, the mutation at position G753 is G753D.

In some embodiments, the amino acid sequence of the variant, relative to SEQ ID NO: 1, comprises:
(1) a mutation at position R34, and any one or two amino acid mutations at positions selected from L170, K172, R173 and F182, wherein the mutation type is selected from a substitution and a deletion, and the mutation at position R34 is R34A; or
(2) a mutation at position R52, and any one amino acid mutation at a position selected from L170 and K179, wherein the mutation type is selected from a substitution and a deletion, and the mutation at position R52 is R52A; or
(3) a mutation at position F55, and any one amino acid mutation at a position selected from L170, Y178, F182 and M183, wherein the mutation type is selected from a substitution and a deletion, and the mutation at position F55 is selected from F55G and F55L; or
(4) a mutation at position L59, and any one or two amino acid mutations at positions selected from K172, R173 and M183, wherein the mutation type is selected from a substitution and a deletion, and the mutation at position L59 is L59A; or
(5) a mutation at position I154, and any one or two amino acid mutations at positions selected from Q232, L446, C510 and C530, wherein the mutation type is selected from a substitution and a deletion, and the mutation at position I154 is I154D; or
(6) a mutation at position L170, and any one amino acid mutation at a position selected from R34, R52, F55 and N871, wherein the mutation type is selected from a substitution and a deletion, and the mutation at position L170 is selected from L170D and L170E; or
(7) mutations at positions K172 and/or R173, and any one amino acid mutation at a position selected from R34, L59, E167, E168, Y178 and K387, wherein the mutation type is selected from a substitution and a deletion, and the mutations at positions K172 and/or R173 are deletions; or mutations at positions K172 and R173, and any one amino acid mutation at a position selected from K387 and K389, wherein the mutation type is selected from a substitution and a deletion, and the mutation at position K172 is K172A and the mutation at position R173 is R173G; or
(8) a mutation at position M183, and any one or two amino acid mutations at positions selected from F55, L59, N171, 1210, R386 and D388, wherein the mutation type is selected from a substitution and a deletion, and the mutation at position M183 is M183E.

In some embodiments, the amino acid sequence of the variant, compared with SEQ ID NO: 1, comprises any one combination of mutations at positions selected from the following:
(1) R34A+Del172-173, R34A+F182E, and R34A+L170E; or
(2) R52A+K179D and R52A+L170E; or
(3) F55G+Y178H, F55L+F182E, F55L+L170E, and F55L+M183E; or
(4) L59A+Del172-173 and L59A+M183E; or
(5) I154D+C530S, 1154D+L446F, and I154D+Q232R+C510Q; or
(6) FSSL+L170E, L170D+N871D, R34A+L170E, and R52A+L170E; or
(7) Del172+E167D, Del172-173+E168D, Del172-173+K387S, Del172-173+Y178G, Del172-173+Y178P, K172A+R173G+K387A, K172A+R173G+K387Q, K172A+R173G+K389A, L59A+Del172-173, and R34A+Del172-173; or
(8) F55L+M183E, L59A+M183E, M183E+I210V, M183E+N171K+D388N, R386H+M183E+I210V, and R386N+M183E+I210V.

In some embodiments, the amino acid sequence of the variant, compared with SEQ ID NO: 1, comprises any one combination of mutations at positions selected from the following: R34A+F182E, R52A+K179D, F55G+Y178H, F55L+F182E, I154D+C530S, I154D+L446F, I154D+Q232R+C510Q, F55L+L170E, L170D+N871D, R34A+L170E, R52A+L170E, Del172+E167D, Del172-173+E168D, Del172-173+K387S, Del172-173+Y178G, Del172-173+Y178P, K172A+R173G+K387A, K172A+R173G+K387Q, K172A+R173G+K389A, L59A+Del172-173, R34A+Del172-173, F55L+M183E, L59A+M183E, M183E+I210V, M183E+N171K+D388N, R386H+M183E+I210V, and R386N+M183E+I210V.

In some embodiments, the amino acid sequence of the variant is as set forth in any one of SEQ ID NOs: 2-58.

In some embodiments, the amino acid sequence of the variant is as set forth in any one of SEQ ID NOs: 3-9, SEQ ID NO: 11, SEQ ID NOs: 15-16, SEQ ID NOs: 19-20, SEQ ID NO: 22 or SEQ ID NOs: 32-58.

### Biological material

The present application provides a polynucleotide encoding an RNA polymerase variant. Due to codon degeneracy and codon usage bias of host cells expressing the polypeptide, the polynucleotide sequence may be any polynucleotide sequence encoding the variant without altering the amino acid sequence thereof. In some embodiments, the polynucleotide sequence encoding the RNA polymerase variant of the present application is as set forth in any one of SEQ ID NOs: 60-116. In some embodiments, the polynucleotide sequence encoding the RNA polymerase variant of the present application is as set forth in any one of SEQ ID NOs: 61-67, SEQ ID NO: 69, SEQ ID NOs: 73-74, SEQ ID NOs: 77-78, SEQ ID NO: 80, and SEQ ID NOs: 90-116.

The expression vector provided by the present application comprises a polynucleotide molecule encoding the RNA polymerase variant of the present application. In some embodiments, the expression vector further comprises one or more regulatory sequences, including but not limited to enhancers, promoters, leader peptide sequences, signal peptide sequences and terminator sequences, wherein the regulatory sequences are operably linked to the polynucleotide molecule encoding the variant.

In some embodiments, the expression vector may be a linear or circular DNA molecule, which generally comprises elements such as a multiple cloning site, a resistance gene and an origin of replication. In some embodiments, the expression vector of the present application is preferably pQE-80L.

The host cell provided by the present application refers to any cell favourable for the expression of the variant of the present application, i.e., any cell susceptible to transformation, transfection or transduction with the expression vector of the present application, and encompasses any progeny cell that differs from the parent cell due to mutations occurring during replication.

In some embodiments, the host cell is a prokaryotic cell, which may be selected from Gram-positive bacteria and Gram-negative bacteria. In some embodiments, the host cell is a Gram-positive bacterium, including but not limited to: *Bacillus*, *Clostridium*, *Enterococcus*, *Geobacillus*, *Lactobacillus*, *Lactococcus*, *Oceanobacillus*, *Staphylococcus*, *Streptococcus* and *Streptomyces*. In some embodiments, the host cell is a Gram-negative bacterium, including but not limited to: *Campylobacter*, *Escherichia coli*, *Flavobacterium*, *Fusobacterium*, *Helicobacter*, *Ilyobacter*, *Neisseria*, *Pseudomonas*, *Salmonella* and *Ureaplasma*. In some embodiments, the host cell is *Escherichia coli* BL21(DE3).

### Method for preparing RNA polymerase variant

The present application provides a method for preparing an RNA polymerase variant, which method comprises: 1) culturing the host cell of the present application under conditions suitable for variant expression; and (2) recovering the variant.

In some embodiments, methods for recovering the variant may be those well known in the art, such as centrifugation, filtration, treatment with a protein-crystallizing precipitant (salting-out), extraction, ultrasonication, ultrafiltration, dialysis, and various chromatography methods such as molecular sieve chromatography (gel filtration), adsorption chromatography, ion exchange chromatography, affinity chromatography and HPLC, and combinations thereof.

In some embodiments, the preparation method further comprises a step of purifying the variant, wherein the purification step can be performed by methods well known in the art, such as chromatography (e.g., ion exchange chromatography, affinity chromatography, hydrophobic interaction chromatography, chromatofocusing and size exclusion chromatography) and ammonium sulphate precipitation.

### Composition

The present application provides a composition comprising at least one RNA polymerase variant of the present application.

The composition of the present application may be a composition for storing the RNA polymerase variant. In some embodiments, in addition to the above RNA polymerase variant, the composition of the present application may optionally comprise components such as buffer components (e.g., Tris base, Tris-HCl, HEPES and MOPS), salts (e.g., NaCl), enzyme inhibitors (e.g., EDTA), reducing agents (e.g., DTT), surfactants (e.g., Triton X-100) and stabilizers (e.g., glycerol). In some embodiments, the composition for storing the RNA polymerase variant of the present application comprises the RNA polymerase variant, Tris-HCl, NaCl, EDTA, DTT, Triton X-100 and glycerol.

In some embodiments, the composition further comprises template DNA. In some embodiments, the composition further comprises at least one *in vitro* transcription component, which may be selected from one or more buffer components, modified or unmodified nucleoside triphosphates, RNase inhibitors, inorganic pyrophosphatase, magnesium ions, etc.

### Kit

The present application provides a kit comprising at least one RNA polymerase variant of the present application.

In some embodiments, the kit may further comprise one or more *in vitro* transcription reaction reagents, such as buffer components, modified or unmodified nucleoside triphosphates, RNase inhibitors, inorganic pyrophosphatase, magnesium ions and water. In some embodiments, the kit further comprises a cap analogue, which may be selected from an unmethylated cap analogue, a dimethylated cap analogue, a trimethylated cap analogue, a dimethylated symmetric cap analogue, and an anti reverse cap analogue. In some embodiments, the kit further comprises at least one *in vitro* transcription component, which may be selected from one or more buffer components, modified or unmodified nucleoside triphosphates, RNase inhibitors, inorganic pyrophosphatase, magnesium ions, etc. In one embodiment, the *in vitro* transcription system component may be selected from a commercially available RNA *in vitro* transcription reagent.

### Use

The present application provides the use of the above RNA polymerase variant, composition or kit in the preparation of RNA by *in vitro* transcription. The present application further provides the use of at least one RNA polymerase variant of the present application for reducing the generation of dsRNA impurities in the preparation of RNA by *in vitro* transcription.

In some embodiments, the preparation of RNA by *in vitro* transcription comprises bringing a DNA template and modified or unmodified nucleoside triphosphates into contact with at least one RNA polymerase variant of the present application, and performing incubation in an *in vitro* transcription reaction system to obtain a target RNA product.

### Preparation method

The present application provides a method for preparing RNA, which comprises bringing a DNA template and modified or unmodified nucleoside triphosphates into contact with at least one RNA polymerase variant of the present disclosure, and performing incubation in an *in vitro* transcription reaction system to obtain a target RNA product.

*In vitro* transcription reaction systems and incubation conditions suitable for generating RNA products are well known in the art. A person of ordinary skill in the art can determine appropriate parameters such as the pH of the reaction system, reaction temperature, reaction time and salt concentration, whether to add exogenous cofactors, etc., in light of the optimal activity of the RNA polymerase. In some embodiments, the *in vitro* transcription reaction system comprises *in vitro* transcription reagents, such as one or more buffer components, modified or unmodified nucleoside triphosphates, RNase inhibitors, inorganic pyrophosphatase, magnesium ions and water. In some embodiments, in the incubation step of the present application, the incubation temperature is 30-50°C, preferably 37°C. In some embodiments, in the incubation step of the present application, the incubation time is 20-240 min, preferably 60 min.

In some embodiments, compared with the RNA product prepared using wild-type RNA polymerase (SEQ ID NO: 1), the RNA product prepared by the method of the present application has a higher yield, and/or higher integrity, and/or lower dsRNA impurity content, and/or more capped mRNA products.

In some embodiments, compared with the RNA product prepared using wild-type RNA polymerase, the RNA product prepared using the solution of the present application has a relative residual amount of dsRNA impurities (ratio of dsRNA residual amount in the variant group to dsRNA residual amount in the wild-type (WT) group) of less than 95%, less than 90%, less than 85%, less than 80%, less than 75%, less than 70%, less than 65%, less than 60%, less than 55%, less than 50%, less than 45%, less than 40%, less than 35%, less than 30%, less than 25%, less than 20%, less than 15%, less than 10%, less than 5%, less than 2%, less than 1% or lower.

In some embodiments, the *in vitro* transcription reaction system comprises one or more buffer components. In some embodiments, the buffer components may be selected from Tris-HCl, Hepes, citric acid or commercially available buffer components. In some embodiments, the *in vitro* transcription buffer system further comprises RNase inhibitors, inorganic pyrophosphatase and magnesium ions. In some embodiments, the *in vitro* transcription buffer system further comprises water (e.g., DEPC-treated water, RNase-free water, DNase-free water, sterile purified water, deionised water, distilled water, etc.). In some embodiments, the *in vitro* transcription reaction system further comprises a cap analogue, which may be selected from an unmethylated cap analogue, a dimethylated cap analogue, a trimethylated cap analogue, a dimethylated symmetric cap analogue, and an anti reverse cap analogue.

### Other embodiments

1. An RNA polymerase variant, wherein the amino acid sequence of the variant, relative to SEQ ID NO: 1, comprises at least one mutation at an amino acid position selected from the following: R34, R52, F55, L59, 1154, E167, E168, L170, N171, K172, R173, Y178, K179, F182, M183, E187, I210, Q232, R386, K387, D388, K389, L446, C510, C530, G753 and N871, wherein the mutation type is selected from a substitution or deletion.
2. The variant of item 1, wherein:
   (1) the substitution at position R34 is A;
   (2) the substitution at position R52 is A;
   (3) the substitution at position F55 is selected from E, L, K, G or N;
   (4) the substitution at position L59 is A;
   (5) the substitution at position I154 is D;
   (6) the substitution at position E167 is D;
   (7) the substitution at position E168 is D;
   (8) the substitution at position L170 is selected from A, D or E;
   (9) the substitution at position N171 is K;
   (10) the substitution at position K172 is A, G or P, or the mutation at position K172 is a deletion;
   (11) the substitution at position R173 is G, or the mutation at position R173 is a deletion;
   (12) the substitution at position Y178 is selected from G, H or P;
   (13) the substitution at position K179 is D;
   (14) the substitution at position F182 is E;
   (15) the substitution at position M183 is E;
   (16) the substitution at position E187 is D;
   (17) the substitution at position I210 is V;
   (18) the substitution at position Q232 is R;
   (19) the substitution at position R386 is selected from H or N;
   (20) the substitution at position K387 is selected from A, Q, S or Y;
   (21) the substitution at position D388 is N;
   (22) the substitution at position K389 is A;
   (23) the substitution at position L446 is F;
   (24) the substitution at position C510 is Q;
   (25) the substitution at position C530 is S;
   (26) the substitution at position G753 is D; and
   (27) the substitution at position N871 is D.
3. The variant of item 1, wherein the amino acid sequence of the variant, relative to SEQ ID NO: 1, comprises any one mutation or combination of mutations at a position or positions selected from the following: R52A, F55E, F55G, F55K, F55L, F55N, L59A, I154D, L170D, M183E, E187D, G753D, R34A+F182E, R52A+K179D, F55G+Y178H, F55L+F182E, 1154D+C530S, I154D+L446F, I154D+Q232R+C510Q, F55L+L170E, L170D+N871D, R34A+L170E, R52A+L170E, Del172+E167D, Del172-173+E168D, Del172-173+K387S, Del172-173+Y178G, Del172-173+Y178P, K172A+R173G+K387A, K172A+R173G+K387Q, K172A+R173G+K389A, L59A+Del172-173, R34A+Del172-173, F55L+M183E, L59A+M183E, M183E+I210V, M183E+N171K+D388N, R386H+M183E+I210V, and R386N+M183E+I210V.
4. The variant of item 1, wherein the amino acid sequence of the variant is as set forth in any one of SEQ ID NOs: 3-9, SEQ ID NO: 11, SEQ ID NOs: 15-16, SEQ ID NOs: 19-20, SEQ ID NO: 22 or SEQ ID NOs: 32-58.
5. A biological material selected from one or more of the following:
   1) a polynucleotide molecule encoding the RNA polymerase variant of any one of items 1-4;
   2) an expression vector comprising the polynucleotide molecule of 1); and
   3) a host cell comprising the polynucleotide molecule of 1) or a host cell comprising the expression vector of 2).
6. A method for preparing the variant of any one of items 1-4, characterized in that the method comprises:
   (1) culturing the host cell of item 5; and
   (2) recovering the variant.
7. A composition comprising the variant of any one of items 1-4.
8. A kit comprising the variant of any one of items 1-4.
9. Use of the variant of any one of items 1-4, the composition of item 7 or the kit of item 8 in *in vitro* transcription.
10. Use of the variant of any one of items 1-4, the composition of item 7 or the kit of item 8 for reducing the generation of dsRNA impurities during the preparation of RNA by *in vitro* transcription.
11. The use of item 10, wherein the preparation of RNA comprises bringing a DNA template into contact with the variant of any one of items 1-4, and performing incubation in an *in vitro* transcription system.
12. A method for preparing RNA, comprising bringing a DNA template and modified or unmodified nucleoside triphosphates into contact with an RNA polymerase variant, and performing incubation in an *in vitro* transcription reaction system to obtain a target RNA product, wherein the amino acid sequence of the variant is as set forth in any one of SEQ ID NOs: 3-9, SEQ ID NO: 11, SEQ ID NOs: 15-16, SEQ ID NOs: 19-20, SEQ ID NO: 22 or SEQ ID NOs: 32-58.

Compared with the prior art, the present application has the following beneficial effects:
Compared with wild-type T7 RNA polymerase, the addition of the T7 RNA polymerase variant of the present application to an *in vitro* transcription reaction system significantly reduces the production of dsRNA contaminants. In particular, the variant combinations F55L+F182E, L170D+N871D, L59A+M183E, M183E+I210V, R34A+F182E, R34A+L170E, R386H+M183E+I210V, R386N+M183E+I210V, R52A+K179D and R52A+L170E can reduce the dsRNA content to less than 0.5% of that of the wild-type polymerase.

### Brief Description of the Drawings

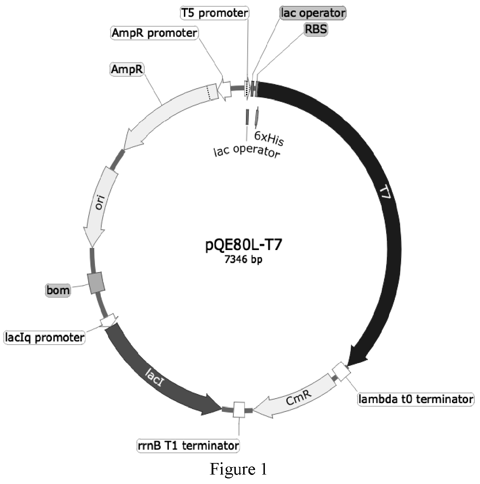

FIG. 1 shows a schematic diagram of the construction of the recombinant plasmid.

### Detailed Description of Embodiments

The technical solutions of the present application are further described below in conjunction with specific examples. However, the following examples are merely illustrative of the present application, and shall not be construed to represent or limit the scope of protection of the present application. The scope of protection of the present application shall be subject to the claims. Unless otherwise specified in the following examples, all reagents and consumables are purchased from ordinary suppliers in the field, and all experimental methods and technical means adopted are conventional methods used in the art.

In the examples of the present application, enzyme activity is defined as follows: under the conditions of 37°C and pH 8.0, the amount of enzyme required to incorporate 1 nmol of [³H] ATP into acid-insoluble precipitates within one hour is defined as one unit of activity.

### Example 1: Preparation of RNA polymerase variants

The RNA polymerase and variants thereof listed in Table 1 (SEQ ID NOs: 60-118) were subjected to DNA sequence synthesis, followed by PCR amplification. The amplified products were then inserted into the BseRI and HindIII restriction sites of the expression vector pQE-80L to construct recombinant expression vectors. The constructed vectors were transformed into *E. coli* BL21(DE3). The bacterial suspension was spread onto an ampicillin-resistant LB agar plate and incubated overnight at 37°C. Single colonies that grew were picked for plasmid extraction and sequencing, and finally recombinant engineered bacteria containing the gene of interest were obtained. The sequence-verified recombinant *E. coli* strains were inoculated into LB medium for overnight activation culture, and then inoculated into fermentation broth (LB medium) at 1-5% V/V. The strains were cultured until the OD 600 value reached 0.6-0.8. IPTG at a final concentration of 0.5 mol/L was then added for further culture for 4-6 h. Subsequently, bacterial cells were collected by centrifugation at 12,000 rpm and 5°C. The collected cells were washed with 0.2 M PBS buffer at pH 7.0 to obtain bacterial pellets. The pellets were disrupted by ultrasonication, followed by purification via affinity chromatography to obtain stock solutions of RNA polymerases.

WT refers to wild-type T7 RNA polymerase, whose amino acid sequence is as follows:

MNTINIAKNDFSDIELAAIPFNTLADHYGERLAREQLALEHESYEMGE
ARFRKMFERQLKAGEVADNAAAKPLITTLLPKMIARINDWFEEVKAKRGK
RPTAFQFLQEIKPEAVAYITIKTTLACLTSADNTTVQAVASAIGRAIEDEARF
GRIRDLEAKHFKKNVEEQLNKRVGHVYKKAFMQVVEADMLSKGLLGGE
AWSSWHKEDSIHVGVRCIEMLIESTGMVSLHRQNAGVVGQDSETIELAPE
YAEAIATRAGALAGISPMFQPCVVPPKPWTGITGGGYWANGRRPLALVRT
HSKKALMRYEDVYMPEVYKAINIAQNTAWKINKKVLAVANVITKWKHCP
VEDIPAIEREELPMKPEDIDMNPEALTAWKRAAAAVYRKDKARKSRRISLE
FMLEQANKFANHKAIWFPYNMDWRGRVYAVSMFNPQGNDMTKGLLTLA
KGKPIGKEGYYWLKIHGANCAGVDKVPFPERIKFIEENHENIMACAKSPLE
NTWWAEQDSPFCFLAFCFEYAGVQHHGLSYNCSLPLAFDGSCSGIQHFSA
MLRDEVGGRAVNLLPSETVQDIYGIVAKKVNEILQADAINGTDNEVVTVT
DENTGEISEKVKLGTKALAGQWLAYGVTRSVTKRSVMTLAYGSKEFGFR
QQVLEDTIQPAIDSGKGLMFTQPNQAAGYMAKLIWESVSVTVVAAVEAM
NWLKSAAKLLAAEVKDKKTGEILRKRCAVHWVTPDGFPVWQEYKKPIQT
RLNLMFLGQFRLQPTINTNKDSEIDAHKQESGIAPNFVHSQDGSHLRKTVV
WAHEKYGIESFALIHDSFGTIPADAANLFKAVRETMVDTYESCDVLADFY
DQFADQLHESQLDKMPALPAKGNLNLRDILESDFAFA (SEQ ID NO: 1)

The corresponding relationship between RNA polymerase and variants thereof and amino acid sequences is shown in Table 1.

**Table 1: Mutation positions of wild-type RNA polymerase and variants thereof and corresponding amino acid sequence numbers**

| SEQ ID NO | Name |
|---|---|
| 1 | WT |
| 2 | R34A |
| 3 | F55E |
| 4 | F55G |
| 5 | F55K |
| 6 | F55L |
| 7 | F55N |
| 8 | G753D |
| 9 | I154D |
| 10 | L170A |
| 11 | Del172 |
| 12 | K172A |
| 13 | K172G |
| 14 | K172P |
| 15 | L170D |
| 16 | L59A |
| 17 | Y178P |
| 18 | Y178G |
| 19 | M183E |
| 20 | R52A |
| 21 | Y178H |
| 22 | E187D |
| 23 | L446F |
| 24 | K387S |
| 25 | K389A |
| 26 | C530S |
| 27 | N871D |
| 28 | Del172-173 |
| 29 | K172A+R173G |
| 30 | K172A+R173G+K387Y |
| 31 | K172A+R173G+K387S |
| 32 | Del172+E167D |
| 33 | Del172-173+E168D |
| 34 | Del172-173+K387S |
| 35 | Del172-173+Y178G |
| 36 | Del172-173+Y178P |
| 37 | F55G+Y178H |
| 38 | F55L+F182E |
| 39 | F55L+L170E |
| 40 | F55L+M183E |
| 41 | I154D+C530S |
| 42 | I154D+L446F |
| 43 | I154D+Q232R+C510Q |
| 44 | K172A+R173G+K387A |
| 45 | K172A+R173G+K387Q |
| 46 | K172A+R173G+K389A |
| 47 | L170D+N871D |
| 48 | L59A+Del172-173 |
| 49 | L59A+M183E |
| 50 | M183E+I210V |
| 51 | M183E+N171K+D388N |
| 52 | R34A+Del172-173 |
| 53 | R34A+F182E |
| 54 | R34A+L170E |
| 55 | R386H+M183E+I210V |
| 56 | R386N+M183E+I210V |
| 57 | R52A+K179D |
| 58 | R52A+L170E |

### Example 2: Generation of dsRNA impurities during in vitro transcription

(1) The enzyme stock solution obtained in Example 1 was diluted with storage buffer (Vazyme, Cat. No. DD4101) to an enzyme activity of 300 U/µL. The reaction components (20 µL) listed in Table 2 were added to an eight-tube strip, mixed thoroughly and centrifuged. The strip was placed in a PCR instrument and incubated at 37°C for 1 h. Then, 36 µL of magnetic beads (Vazyme, Cat. No. N412) were added, mixed thoroughly and incubated at room temperature for 2-5 min. The mixture was placed on a magnetic rack to purify RNA products, which were then transferred to RNase-free centrifuge tubes to obtain purified RNA products.
(2) The content of dsRNA impurities was determined using a dsRNA detection kit (Vazyme, Cat. No. DD3509).

**Table 2: Reaction system composition**

| Component | Concentration | Volume (µL) |
|---|---|---|
| ATP | 100 mM | 2 |
| GTP | 100 mM | 2 |
| CTP | 100 mM | 2 |
| UTP | 100 mM | 2 |
| RNase inhibitor (Vazyme, Cat. No. GMP4102PA) | 40 U/µL | 1 |
| Inorganic pyrophosphatase (Vazyme, Cat. No. GMP4103PC) | 0.1 U/µL | 1 |
| Template DNA (SEQ ID NO: **117**) | \ | 1 µg |
| 10× reaction buffer (Vazyme, Cat. No. DD4101R) | \ | 2 |
| T7 RNA polymerase | 300 U/µL | 1 |
| ddH2O | \ | Make up to 20 µL |

**Table 3: dsRNA detection results**

| SEQ ID NO | Name | Relative residual rate of dsRNA (%) |
|---|---|---|
| 1 | WT | 100 |
| 2 | R34A | 89.8 |
| 3 | F55E | 11.09 |
| 4 | F55G | 2.39 |
| 5 | F55K | 5.86 |
| 6 | F55L | 47.72 |
| 7 | F55N | 5.27 |
| 8 | G753D | 7.98 |
| 9 | I154D | 6.54 |
| 10 | L170A | 44.50 |
| 11 | Del172 | 32.42 |
| 12 | K172A | 49.9 |
| 13 | K172G | 27.93 |
| 14 | K172P | 7.83 |
| 15 | L170D | 2.51 |
| 16 | L59A | 65.28 |
| 17 | Y178P | 93.94 |
| 18 | Y178G | > 100 |
| 19 | M183E | 2.91 |
| 20 | R52A | 52.42 |
| 21 | Y178H | 19.18 |
| 22 | E187D | 3.81 |
| 23 | L446F | > 100 |
| 24 | K387S | 61.35 |
| 25 | K389A | 80.6 |
| 26 | C530S | > 100 |
| 27 | N871D | > 100 |
| 28 | Del172-173 | 3.12 |
| 29 | K172A+R173G | 7.42 |
| 30 | K172A+R173G+K387Y | 7.17 |
| 31 | K172A+R173G+K387S | 3.98 |
| 32 | Del172+E167D | 8.06 |
| 33 | Del172-173+E168D | 2.55 |
| 34 | Del172-173+K387S | 1.47 |
| 35 | Del172-173+Y178G | 1.67 |
| 36 | Del172-173+Y178P | 1.94 |
| 37 | F55G+Y178H | 1.98 |
| 38 | F55L+F182E | 0.92 |
| 39 | F55L+L170E | 1.55 |
| 40 | F55L+M183E | 1.38 |
| 41 | I154D+C530S | 3.48 |
| 42 | I154D+L446F | 2.45 |
| 43 | I154D+Q232R+C510Q | 5.96 |
| 44 | K172A+R173G+K387A | 2.42 |
| 45 | K172A+R173G+K387Q | 2.37 |
| 46 | K172A+R173G+K389A | 2.6 |
| 47 | L170D+N871D | 0.58 |
| 48 | L59A+Del172-173 | 1.46 |
| 49 | L59A+M183E | 0.53 |
| 50 | M183E+I210V | 0.75 |
| 51 | M183E+N171K+D388N | 1.2 |
| 52 | R34A+Del172-173 | 1.08 |
| 53 | R34A+F182E | 0.9 |
| 54 | R34A+L170E | 1 |
| 55 | R386H+M183E+I210V | 0.66 |
| 56 | R386N+M183E+I210V | 0.75 |
| 57 | R52A+K179D | 0.68 |
| 58 | R52A+L170E | 0.78 |

The dsRNA detection results are shown in Table 3. Compared with the WT group, all polymerase variants obtained in Example 1 could effectively reduce the generation of dsRNA impurities during *in vitro* transcription. Among these, the mutant combinations F55L+F182E, L170D+N871D, L59A+M183E, M183E+I210V, R34A+F182E, R34A+L170E, R386H+M183E+I210V, R386N+M183E+I210V, R52A+K179D and R52A+L170E could reduce the dsRNA content to less than 1% of that of the wild-type polymerase.

## Claims

1. An RNA polymerase variant, **characterized in that** the amino acid sequence of the variant, relative to SEQ ID NO: 1, comprises at least one mutation at amino acid positions selected from the following: R34, R52, F55, L59, I154, L170, K172, R173, M183, E187 and G753, wherein the mutation type is selected from a substitution and a deletion.

2. The variant of claim 1, **characterized in that** the amino acid sequence of the variant has one amino acid mutation relative to SEQ ID NO: 1, and the position of the mutated amino acid is selected from R52, F55, L59, I154, L170, K172, M183, E187 and G753.

3. The variant of claim 2, **characterized in that**:
(1) the mutation at position R52 is R52A;
(2) the mutation at position F55 is selected from F55E, F55G, F55K, F55L and F55N;
(3) the mutation at position L59 is L59A;
(4) the mutation at position I154 is I154D;
(5) the mutation at position L170 is selected from L170A and L170D;
(6) the mutation at position K172 is a deletion;
(7) the mutation at position M183 is M183E;
(8) the mutation at position E187 is E187D; and
(9) the mutation at position G753 is G753D.

4. The variant of claim 1, **characterized in that** the amino acid sequence of the variant, relative to SEQ ID NO: 1, comprises:
(1) a mutation at position R34, and any one or two amino acid mutations at positions selected from L170, K172, R173 and F182, wherein the mutation type is selected from a substitution and a deletion, and the mutation at position R34 is R34A; or
(2) a mutation at position R52, and any one amino acid mutation at a position selected from L170 and K179, wherein the mutation type is selected from a substitution and a deletion, and the mutation at position R52 is R52A; or
(3) a mutation at position F55, and any one amino acid mutation at a position selected from L170, Y178, F182 and M183, wherein the mutation type is selected from a substitution and a deletion, and the mutation at position F55 is selected from F55G and F55L; or
(4) a mutation at position L59, and any one or two amino acid mutations at positions selected from K172, R173 and M183, wherein the mutation type is selected from a substitution and a deletion, and the mutation at position L59 is L59A; or
(5) a mutation at position I154, and any one or two amino acid mutations at positions selected from Q232, L446, C510 and C530, wherein the mutation type is selected from a substitution and a deletion, and the mutation at position I154 is I154D; or
(6) a mutation at position L170, and any one amino acid mutation at a position selected from R34, R52, F55 and N871, wherein the mutation type is selected from a substitution and a deletion, and the mutation at position L170 is selected from L170D and L170E; or
(7) mutations at positions K172 and/or R173, and any one amino acid mutation at a position selected from R34, L59, E167, E168, Y178 and K387, wherein the mutation type is selected from a substitution and a deletion, and the mutations at positions K172 and/or R173 are deletions; or mutations at positions K172 and R173, and any one amino acid mutation at a position selected from K387 and K389, wherein the mutation type is selected from a substitution and a deletion, and the mutation at position K172 is K172A and the mutation at position R173 is R173G; or
(8) a mutation at position M183, and any one or two amino acid mutations at positions selected from F55, L59, N171, I210, R386 and D388, wherein the mutation type is selected from a substitution and a deletion, and the mutation at position M183 is M183E.

5. The variant of claim 4, **characterized in that** the amino acid sequence of the variant, relative to SEQ ID NO: 1, comprises any one combination of mutations at positions selected from the following:
R34A+F182E, R52A+K179D, F55G+Y178H, F55L+F182E, I154D+C530S, I154D+L446F, I154D+Q232R+C510Q, F55L+L170E, L170D+N871D, R34A+L170E, R52A+L170E, Del172+E167D, Del172-173+E168D, Del172-173+K387S, Del172-173+Y178G, Del172-173+Y178P, K172A+R173G+K387A, K172A+R173G+K387Q, K172A+R173G+K389A, L59A+Del172-173, R34A+Del172-173, F55L+M183E, L59A+M183E, M183E+I210V, M183E+N171K+D388N, R386H+M183E+I210V, and R386N+M183E+I210V.

6. The variant of claim 1, **characterized in that** the amino acid sequence of the variant is as set forth in any one of SEQ ID NOs: 2-58.

7. A biological material, **characterized in that** the biological material is selected from one or more of the following:
1) a polynucleotide molecule encoding the RNA polymerase variant of any one of claims 1-6;
2) an expression vector comprising the polynucleotide molecule of 1); and
3) a host cell comprising the polynucleotide molecule of 1) or a host cell comprising the expression vector of 2).

8. A method for preparing the variant of any one of claims 1-6, **characterized in that** the method comprises:
(1) culturing the host cell of claim 7; and
(2) recovering the variant.

9. A composition, **characterized in that** the composition comprises the variant of any one of claims 1-6.

10. A kit, **characterized in that** the kit comprises the variant of any one of claims 1-6.

11. Use of the variant of any one of claims 1-6, the composition of claim 9 or the kit of claim 10 in *in vitro* transcription.

12. Use of the variant of any one of claims 1-6, the composition of claim 9 or the kit of claim 10 for reducing the generation of dsRNA impurities during RNA preparation by *in vitro* transcription.

13. The use of claim 12, **characterized in that** the preparation of RNA comprises bringing a DNA template into contact with the variant of any one of claims 1-6 and performing incubation in an *in vitro* transcription system.

14. A method for preparing RNA, **characterized in that** the method comprises bringing a DNA template and modified or unmodified nucleoside triphosphates into contact with an RNA polymerase variant, and performing incubation in an *in vitro* transcription reaction system to obtain a target RNA product, wherein the amino acid sequence of the RNA polymerase variant is as set forth in any one of SEQ ID NOs: 2-58.
